(19) Europäisches Patentamt
European Patent Office
Office européen des brevets

(11) EP 0 626 380 B1

(12) EUROPÄISCHE PATENTSCHRIFT

(45) Veröffentlichungstag und Bekanntmachung des Hinweises auf die Patenterteilung:
**13.01.1999 Patentblatt 1999/02**

(21) Anmeldenummer: **94107472.6**

(22) Anmeldetag: **13.05.1994**

(51) Int. Cl.$^6$: **C07D 285/12**, C07D 277/68, C07D 263/58, C07D 285/08, C07D 277/56, A01N 43/76, A01N 43/78, A01N 43/82

# (54) N-(4-Fluorphenyl)-heteroaryloxyacetamide als Herbizide

N-(4-Fluorophenyl)-heteroaryloxyacetamide as herbicides

N-(4-Fluorophényl)-hétéroaryloxyacétamides comme herbicides

(84) Benannte Vertragsstaaten:
**BE CH DE ES FR GB IT LI NL**

(30) Priorität: **25.05.1993 DE 4317323**

(43) Veröffentlichungstag der Anmeldung:
**30.11.1994 Patentblatt 1994/48**

(73) Patentinhaber: **BAYER AG**
**51368 Leverkusen (DE)**

(72) Erfinder:
- **Förster, Heinz Dr.**
  **D-56337 Kadenbach (DE)**
- **Santel, Hans-Joachim Dr.**
  **D-51371 Leverkusen (DE)**
- **Dollinger, Markus Dr.**
  **D-51381 Leverkusen (DE)**

(56) Entgegenhaltungen:
**EP-A- 0 037 524**
**EP-A- 0 443 164**
**EP-A- 0 510 479**
**EP-A- 0 537 539**
**EP-A- 0 572 893**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

**Beschreibung**

Die Erfindung betrifft neue N-(4-Fluor-phenyl)-heteroaryloxyacetamide, ein Verfahren zu ihrer Herstellung und ihre Verwendung als Herbizide.

Es ist bereits bekannt, daß bestimmte Heteroaryloxyacetamide, wie z.B. N-Methyl-2-(5-chlor-1,3,4-thiadiazol-2-yl-oxy)-acetanilid (vgl. EP-A 192117), herbizide Eigenschalten aufweisen (vgl. auch EP-A 5501, EP-A 18497, EP-A 37524, EP-A 94541. EP-A 217496, EP-A 348735, EP-A 348736, EP-A 348737).

Die herbizide Wirksamkeit der vorbekannten Heteroaryloxyacetamide ist jedoch nicht immer ganz zufriedenstellend.

Es wurden nun die neuen N-(4-Fluor-phenyl)-heteroaryloxyacetamide der allgemeinen Formel (I) gefunden,

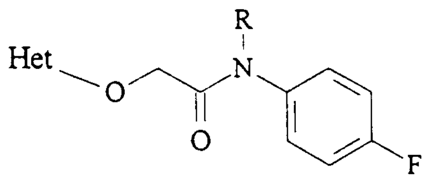

(I)

in welcher

R für Methyl oder Ethyl steht und

Het für einen aromatischen Fünfring-Heterocyclus aus der Reihe Oxazolyl oder Thiazolyl (welche jeweils gegebenenfalls durch Halogen, Cyano, Methyl, Ethyl, n- oder i-Propyl, n-, i-, s- oder t-Butyl, Difluormethyl, Dichlormethyl, Trifluormethyl oder Trichlormethyl substituiert sind), 1,2,4-Oxadiazolyl, 1,3,4-Oxadiazolyl, 1,2,4-Thiadiazolyl oder 1,3,4-Thiadiazolyl (welche jeweils gegebenenfalls durch Fluor, Chlor, Brom, Methyl, Ethyl, n- oder i-Propyl, n-, i-, s-oder t-Butyl, Trifluormethyl, Dichlormethyl, Trichlormethyl, Chlordifluormethyl, Fluordichlormethyl, Methylthio, Ethylthio, n- oder i-Propylthio, Methylsulfinyl, Ethylsulfinyl, n- oder i-Propylsulfinyl, Methylsulfonyl, Ethylsulfonyl, n-oder i-Propylsulfonyl, Phenyl oder Fluorphenyl substituiert sind), Benzoxazolyl oder Benzthiazolyl (welche jeweils durch Chlor, Brom, Methyl, Ethyl, n-oder i-Propyl, Difluormethyl, Dichlormethyl, Trifluormethyl oder Trichlormethyl substituert sind) und - für den Fall, daß R für Ethyl steht - auch für unsubstituiertes Benzoxazolyl und Benzthiazolyl steht.

Weiter wurde gefunden, daß man die neuen Verbindungen der Formel (I) erhält, wenn man Heterocyclen der allgemeinen Formel (II)

Het-X (II)

in welcher

Het die oben angegebene Bedeutung hat und

X für Halogen oder Alkylsulfonyl steht,

mit N-(4-Fluor-phenyl)-hydroxyacetamiden der allgemeinen Formel (III)

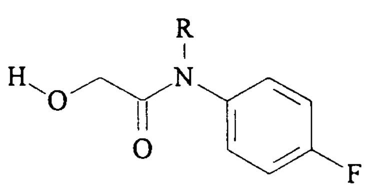

(III)

in welcher

R die oben angegebene Bedeutung hat,

gegebenenfalls in Gegenwart eines Säureakzeptors, gegebenenfalls in Gegenwart eines Verdünnungsmittels und gegebenenfalls in Gegenwart eines Katalysators umsetzt.

Ferner wurde gefunden, daß die neuen N-(4-Fluor-phenyl)-heteroaryloxyacetamide der allgemeinen Formel (I) interessante herbizide Eigenschaften besitzen.

Überraschenderweise zeigen die erfindungsgemäßen Verbindungen der Formel (I) bei sehr guter Verträglichkeit gegenüber Kulturpflanzen, wie z.B. Soja, erheblich stärkere Wirkung gegen Unkräuter als die bekannte chemisch ähnliche Verbindung N-Methyl-2-(5-chlor-1,3,4-thladiazol-2-yl-oxy)-acetanilid.

Die Erfindung betrifft insbesondere Verbindungen der Formel (I), in welcher

R für Methyl oder Ethyl steht und

Het für 1,2,4-Thiadiazolyl (welches gegebenenfalls durch Chlor, Trifluormethyl, Chlordifluormethyl oder Methylsulfonyl substituiert ist), 1,3,4-Thiadiazolyl (welches gegebenenfalls durch Chlor, Trifluormethyl, Chlordifluormethyl oder Methylsulfonyl substituiert ist), oder für jeweils einfach durch Chlor substituiertes Benzoxazolyl oder Benzthiazolyl steht.

Die oben aufgeführten allgemeinen oder im Vorzugsbereich angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise 3,5-Dichlor-1,2,4-thiadiazol und N-(4-Fluor-phenyl)-N-methyl-hydroxyacetamid als Ausgangsstoffe, so läßt sich der Reaktionsablauf beim erfindungsgemäßen Herstellungsverfahren durch das folgende Formelschema darstellen:

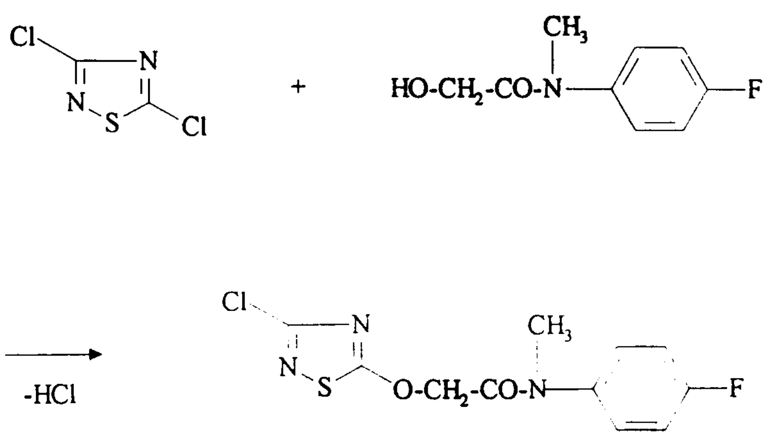


Die als Ausgangsstoffe zu verwendenden Heterocyclen der allgemeinen Formel (II) sind bekannt und/oder können nach an sich bekannten Vefahren hergestellt werden (vgl. J. Org. Chem. 27 (1962), 2589-2592; EP-A 18497; EP-A 165537; EP-A 308740; EP-A 348735; EP-A 348737).

Die weiter als Ausgangsstoffe zu verwendenden N-(4-Fluor-phenyl)-hydroxyacetamide der Formel (III) sind ebenfalls bekannt und/oder können nach an sich bekannten Verfahren hergestellt werden (vgl. EP-A 37524; EP-A 348735; Herstellungsbeispiele).

Das erfindungsgemäße Verfahren zu Herstellung der neuen Verbindungen der Formel (I) wird vorzugsweise unter Verwendung von Verdünnungsmitteln durchgeführt. Hierzu gehören vorzugsweise Kohlenwasserstoffe, wie z.B. Toluol, Xylol oder Cyclohexan, Halogenkohlenwasserstoffe, wie z.B. Methylenchlorid, Ethylenchlorid, Chloroform oder Chlorbenzol, Ether, wie z.B. Diethylether, Dipropylether, Diisopropylether, Dibutylether, Diisobutylether, Glycoldimethylether, Tetrahydrofuran und Dioxan, Alkohole, wie z.B. Methanol, Ethanol, Propanol, Isopropanol, Butanol, Isobutanol, sec-Butanol oder tert-Butanol, Ketone, wie z.B. Aceton, Methylethylketon, Methylisopropylketon und Methylisobutylketon, Ester, wie z.B. Essigsäuremethylester und Essigsäureethylester, Amide, wie z.B. Dimethylformamid, Dimethylacetamid und N-Methyl-pyrrolidon, Nitrile, wie z.B. Acetonitril und Propionitril, Sulfoxide, wie z.B. Dimethylsulfoxid sowie Wasser

oder wäßrige Salzlösungen.

Als Salze verwendet man hierbei vorzugsweise Chloride oder Sulfate von Alkali-oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhalt unter Verwendung von Säurebindemitteln durchgeführt. Als solche werden vorzugsweise stark basische Alkali- und Erdalkalimetallverbindungen, beispielsweise Oxide, wie z.B. Natrium-, Kalium-, Magnesium- und Calciumoxid, Hydroxide, wie z.B. Natrium-, Kalium-, Magnesium- und Calciumhydroxid, Alkoholate, wie z.B. Natrium- und Kalium-tert-butylat und/oder Carbonate, wie z.B. Natrium-, Kalium-, Magnesium- und Calciumcarbonat verwendet.

Der Zusatz von 0,01 bis 10 Gew.-% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:

Tetrabutylammoniumchlorid, Tetrabutylammoniumbromid, Tributyl-methylphosphoniumbromid, Trimethyl-$C_{13}/C_{15}$-alkylammoniumchlorid, Dibenzyl-dimethylammonium-methylsulfat, Dimethyl-$C_{12}/C_{14}$-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50°C und +110°C, vorzugsweise bei Temperaturen zwischen -20°C und +80°C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol Heterocyclus der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cypetus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämptung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in dikotylen Kulturen, wie z.B. in Soja, vor allem im Vorauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Ke-phaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin; Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

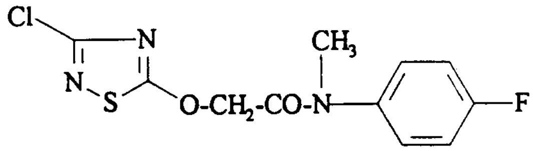

3,6 g (20 mMol) 3,5-Dichlor-1,2,4-thiadiazol werden zusammen mit 3,9 g (20 mMol) N-(4-Fluor-phenyl)-N-methyl-hydroxyacetamid in 70 ml Aceton vermischt und auf -20°C abgekühlt. Bei dieser Temperatur wird dann eine Lösung von 0,92 g (23 mMol) Natriumhydroxid in 5 ml Wasser tropfenweise zur Mischung gegeben. Nach Entfernen der Kühlung wird dann das Reaktionsgemisch 12 Stunden, zuletzt bei 20°C, gerührt.

Zur Aufarbeitung wird mit 2N-Salzsäuue angesäuert und dann mit Wasser weiter auf etwa das doppelte Volumen verdünnt. Dann wird mit Chloroform extrahiert, die organische Phase mit Natriumsulfat getrocknet und filtriert. Vom Filtrat wird das Lösungsmittel im Wasserstrahlvakuum sorgfältig abdestilliert.

Man erhalt 4,8 g (80% der Theorie) N-(4-Fluor-phenyl)-N-methyl-2-(3-chlor-1,2,4-thiadiazol-5-yl-oxy)-acetamid als öligen Rückstand vom Brechungsindex $n_D^{20}$ = 1,5468.

Analog Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

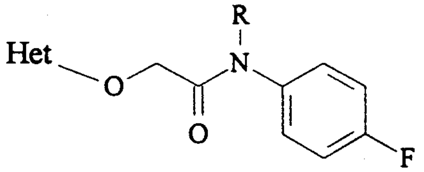

(I)

Tabelle 1:

Beispiele für die Verbindungen der Formel (I)

| Bsp.-Nr. | Het. | R | Physikal. Daten |
|---|---|---|---|
| 2 | 2-Trifluormethyl-1,3,4-thiadiazol-5-yl | Methyl | Fp.: 51°C |
| 3 | 3-Trifluormethyl-1,2,4-thiadiazol-5-yl | Methyl | Fp.: 35°C |
| 4 | 6-Chlor-benzoxazol-2-yl | Methyl | Fp.: 150°C |
| 5 | 2-Chlordifluormethyl-1,3,4-thiadiazol-5-yl | Methyl | $n_D^{20}$ = 1,5178 |
| 6 | 3-Methylsulfonyl-1,2,4-thiadiazol-5-yl | Methyl | $n_D^{20}$ = 1,5402 |
| 7 | 2-Methylsulfonyl-1,3,4-thiadiazol-5-yl | Methyl | $n_D^{20}$ = 1,5524 |
| 8 | 2-Fluordichlormethyl-1,3,4-thiadiazol-5-yl | Methyl | $n_D^{20}$ = 1,5495 |

Tabelle 1: (Fortsetzung)

| Bsp.-Nr. | Het. | R | Physikal. Daten |
|---|---|---|---|
| 9 | 3-Methylsulfinyl-1,2,4-thiadiazol-5-yl | Methyl | $n_D^{20} = 1,5648$ |
| 10 | 4-Chlor-benzthiazol-2-yl | Methyl | Fp.: 113°C |
| 11 | 2-Chlor-1,3,4-thiadiazol-5-yl | Methyl | Fp.: 89°C |
| 12 | 2-(2-Fluor-phenyl)-1,3,4-thiadiazol-5-yl | Methyl | Fp.: 133°C |
| 13 | 3-Trichlormethyl-1,2,4-thiadiazol-5-yl | Methyl | Fp.: 88°C |
| 14 | 5-Methyl-benzoxazol-2-yl | Methyl | Fp.: 133°C |
| 15 | 6-Methyl-benzthiazol-2-yl | Methyl | Fp.: 77°C |
| 16 | 5-Cyano-4-methyl-thiazol-2-yl | Methyl | Fp.: 109°C |
| 17 | 3-Chlor-1,2,4-oxadiazol-5-yl | Methyl | Fp.: 73°C |
| 18 | 3-Methyl-1,2,4-thiadiazol-5-yl | Methyl | Fp.: 80°C |
| 19 | 3-Methylthio-1,2,4-thiadiazol-5-yl | Methyl | $n_D^{20} = 1,5672$ |

Tabelle 1: (Fortsetzung)

| Bsp.-Nr. | Het. | R | Physikal. Daten |
|---|---|---|---|
| 20 | 3-Isopropyl-1,2,4-thiadiazol-5-yl | Methyl | $n_D^{20}$ = 1,5345 |
| 21 | 3-Propyl-1,2,4-thiadiazol-5-yl | Methyl | Fp.: 53°C |
| 22 | 5-Trifluormethyl-benzthiazol-2-yl | Methyl | Fp.: 81°C |
| 23 | 3-Methylsulfonyl-1,2,4-thiadiazol-5-yl | Ethyl | Fp.: 98°C |
| 24 | 6-Chlor-benzoxazol-2-yl | Ethyl | Fp.: 115°C |
| 25 | 2.Fluordichlormethyl-1,3,4-thiadiazol-5-yl | Ethyl | Fp.: 57°C |
| 26 | 3-Trifluormethyl-1,2,4-thiadiazol-5-yl | Ethyl | Fp.: 35°C |
| 27 | 2-Trifluormethyl-1,3,4-thiadiazol-5-yl | Ethyl | Fp.: 61°C |
| 28 | Benzoxazol-2-yl | Ethyl | Fp.: 98°C |
| 29 | 2-Chlordifluormethyl-1,3,4-thiadiazol-5-yl | Ethyl | Fp.: 62°C |
| 30 | 3-Methylsulfinyl-1,2,4-thiadiazol-5-yl | Ethyl | $n_D^{20}$ = 1,5609 |
| 31 | Benzthiazol-2-yl | Ethyl | Fp.: 76°C |

Tabelle 1: (Fortsetzung)

| Bsp.-Nr. | Het. | R | Physikal. Daten |
|---|---|---|---|
| 32 | 2-Methylsulfonyl-1,3,4-thiadiazol-5-yl | Ethyl | $n_D^{20} = 1{,}5458$ |
| 33 | 2-tert-Butyl-oxadiazol-5-yl | Ethyl | $n_D^{20} = 1{,}4929$ |
| 34 | 2-(2-Fluor-phenyl)-1,3,4-thiadiazol-5-yl | Ethyl | Fp.: 127°C |
| 35 | 5-Methyl-benzoxazol-2-yl | Ethyl | Fp.: 125°C |
| 36 | 5-Cyano-4-methyl-thiazol-2-yl | Ethyl | Fp.: 83°C |
| 37 | 2-Chlor-1,3,4-thiadiazol-2-yl | Ethyl | Fp.: 76°C |
| 38 | 6-Chlor-benzthiazol-2-yl | Ethyl | Fp.: 92°C |
| 39 | 3-Trichlormethyl-1,2,4-thiadiazol-5-yl | Ethyl | Fp.: 58°C |
| 40 | 3-Isopropyl-1,2,4-thiadiazol-5-yl | Ethyl | $n_D^{20} = 1{,}5288$ |
| 41 | 5-Chlor-benzoxazol-2-yl | Methyl | Fp.: 110°C |
| 42 | 5-Chlor-benzoxazol-2-yl | Ethyl | Fp.: 122°C |
| 43 | 3-Chlor-1,2,4-oxadiazol-5-yl | Ethyl | Fp.: 51°C |

Tabelle 1: (Fortsetzung)

| Bsp.-Nr. | Het. | R | Physikal. Daten |
|---|---|---|---|
| 44 | 3-Methyl-1,2,4-thiadiazol-5-yl | Ethyl | $n_D^{20} = 1{,}5321$ |
| 45 | 3-Methylthio-1,2,4-thiadiazol-5-yl | Ethyl | $n_D^{20} = 1{,}5675$ |
| 46 | 3-Propyl-1,2,4-thiadiazol-5-yl | Ethyl | $n_D^{20} = 1{,}5208$ |
| 47 | 3-Chlor-1,2,4-thiadiazol-5-yl | Ethyl | $n_D^{20} = 1{,}5538$ |
| 48 | 3-Ethylsulfonyl-1,2,4-thiadiazol-5-yl | Methyl | $n_D^{20} = 1{,}5392$ |
| 49 | 3-Ethylsulfonyl-1,2,4-thiadiazol-5-yl | Ethyl | Fp.: 78°C |

Ausgangsstoffe der Formel (III):

Beispiel (III-1)

1. Stufe:

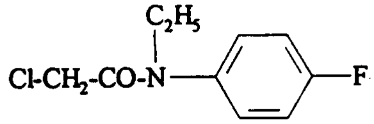

28 g (0,2 Mol) N-Ethyl-4-fluor-anilin (Herstellung vgl. Synthesis 1979, 727-729) in 400 ml Methylenchlorid werden mit 27,6 g Kaliumcarbonat in 60 ml Wasser verrührt und zu diesem Zweiphasensystem werden bei 0°C bis 5°C unter

Rühren 22,5 g (0,2 Mol) Chloracetylchlorid gegeben. Nach Entfernen der Kühlung läßt man das Reaktionsgemisch 12 Stunden, zuletzt bei 20°C rühren, verdünnt dann mit Wasser auf etwa das doppelte Volumen. Die organische Phase wird dann abgetrennt, mit Natriumsulfat getrocknet und filtriert. Vom Filtrat wird das Lösungsmittel im Wasserstrahlvakuum abdestilliert.

Man erhält 42 g (98% der Theorie) N-(4-Fluorphenyl)-N-ethyl-chloracetamid als öligen Rückstand vom Brechungsindex $n_D^{20}$ = 1,5196.

2. Stufe:

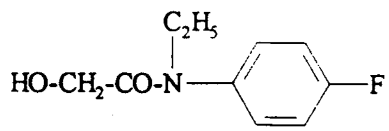

Eine Mischung aus 370 g (1,72 Mol) N-(4-Fluor-phenyl)-N-ethyl-chloracetamid, 52 g Kaliumcarbonat und 1,9 ml Triethylamin wird auf 120°C erhitzt. Dann werden innerhalb von 2 Stunden 180 g Natriumacetat portionsweise dazugegeben und das Gemisch wird noch etwa eine Stunde bei 120°C gerührt. Nach Abkühlen auf ca. 50°C läßt man 970 ml Methanol in die Mischung einlaufen und erhitzt noch 4 Stunden unter Rückfluß. Dann wird eingeengt, der Rückstand mit ca. 1 Liter Wasser verrührt und das kristallin angefallene Produkt durch Absaugen isoliert.

Man erhält 315 g (93% der Theorie) N-(4-Fluor-phenyl)-N-ethyl-hydroxyacetamid vom Schmelzpunkt 66°C.

Anwendungsbeispiele:

In den nachfolgenden Anwendungsbeispielen wird die nachstehend aufgeführte Verbindung als Vergleichssubstanz herangezogen:

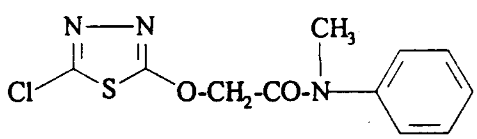

(A)

N-Methyl-2-(5-Chlor-1,3,4-thiadiazol-2-yl-oxy)-acetanilid
(bekannt aus EP-A 192117).

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

O % = keine Wirkung (wie unbehandelte Kontrolle)

100 % = totale Vernichtung

Eine deutliche Überlegenheit in der Wirksamkeit gegenüber dem Stand der Technik zeigen in diesem Test z.B. die Verbindungen gemäß folgender Herstellungsbeispiele: 2, 3, 4, 5, 6, 8, 9 und 11.

Tabelle A: Pre-emergence-Test/Gewächshaus

| Wirkstoff | Aufwand-menge (g/ha) | So-ja | Digi-ta-ria | Poa | Seta-ria |
|---|---|---|---|---|---|
| 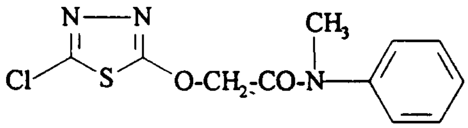 (A) (bekannt) | 125 | 0 | 50 | 40 | 70 |
| 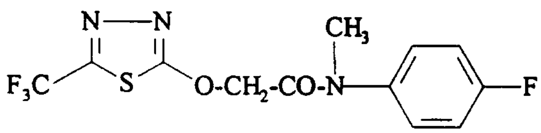 (2) | 125 | 0 | 100 | 90 | 100 |
| 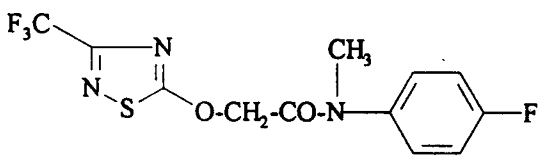 (3) | 125 | 0 | 100 | 95 | 90 |
| 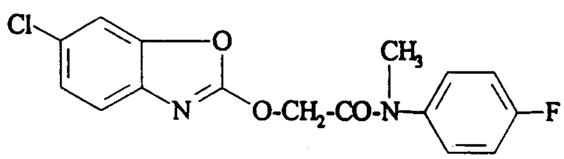 (4) | 125 | 0 | 90 | - | 95 |
| 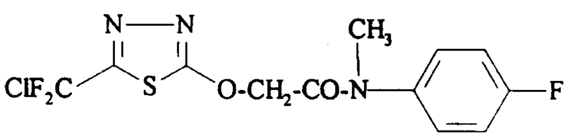 (5) | 125 | 0 | 95 | 90 | 95 |

Tabelle A: (Fortsetzung)

| Wirkstoff | Aufwand-menge (g/ha) | So-ja | Digi-ta-ria | Poa | Seta-ria |
|---|---|---|---|---|---|
| 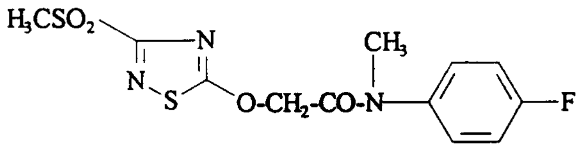 (6) | 125 | 0 | 100 | 80 | 100 |
| 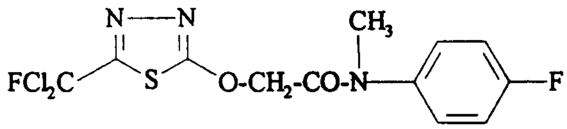 (8) | 125 | 0 | 90 | 80 | 90 |
| 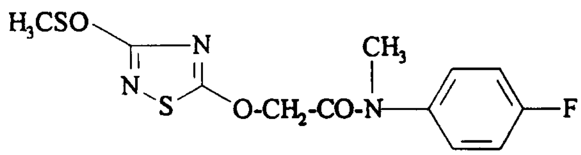 (9) | 125 | 10 | 95 | - | 95 |
| 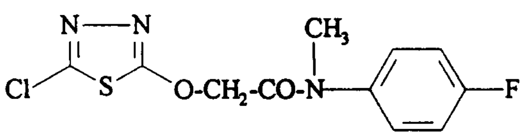 (11) | 125 | 10 | 100 | 100 | 100 |

**Patentansprüche**

1. N-(4-Fluor-phenyl)-heteroaryloxyacetamide der allgemeinen Formel (I),

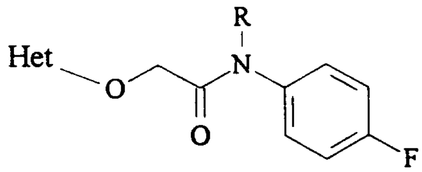

(I)

in welcher

R für Methyl oder Ethyl steht und

Het für einen aromatischen Fünfring-Heterocyclus aus der Reihe Oxazolyl oder Thiazolyl (welche jeweils gegebenenfalls durch Halogen, Cyano, Methyl, Ethyl, n- oder i-Propyl, n-, i-, s- oder t-Butyl, Difluormethyl, Dichlormethyl, Trifluormethyl oder Trichlormethyl substituiert sind), 1,2,4-Oxadiazolyl, 1,3,4-Oxadiazolyl, 1,2,4-Thiadiazolyl oder 1,3,4-Thiadiazolyl (welche jeweils gegebenenfalls durch Fluor, Chlor, Brom, Methyl, Ethyl, n- oder i-Propyl, n-, i-, s- oder t-Butyl, Trifluormethyl, Dichlormethyl, Trichlormethyl, Chlordifluormethyl, Fluordichlormethyl, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Phenyl oder Fluorphenyl substituiert sind), Benzoxazolyl oder Benzthiazolyl (welche jeweils durch Chlor, Brom, Methyl, Ethyl, n-oder i-Propyl, Difluormethyl, Dichlormethyl, Trifluormethyl oder Trichlormethyl substituert sind) und - für den Fall, daß R für Ethyl steht - auch für unsubstituiertes Benzoxazolyl und Benzthiazolyl steht.

**2.** N-(4-Fluor-phenyl)-heteroaryloxyacetamide der Formel (I) gemäß Anspruch 1, dadurch gekennzeichnet, daß darin

R für Methyl oder Ethyl steht und

Het für 1,2,4-Thiadiazolyl (welches gegebenenfalls durch Chlor, Trifluormethyl, Chlordifluormethyl oder Methylsulfonyl substituiert ist), 1,3,4-Thiadiazolyl (welches gegebenenfalls durch Chlor, Trifluormethyl, Chlordifluormethyl oder Methylsulfonyl substituiert ist), oder für jeweils einfach durch Chlor substituiertes Benzoxazolyl oder Benzthiazolyl steht.

**3.** Verfahren zur Herstellung von N-(4-Fluorphenyl)-heteroaryloxyacetamiden der Formel (I) gemäß Anspruch 1, dadurch gekennzeichnet, daß man Heterocyclen der allgemeinen Formel (II)

Het-X (II)

in welcher

Het die in Anspruch 1 angegebene Bedeutung hat und

X für Halogen oder Alkylsulfonyl steht,

mit N-(4-Fluor-phenyl) -hydroxyacetamiden der allgemeinen Formel (III)

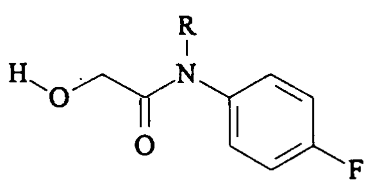

(III)

in welcher

R die in Anspruch 1 angegebene Bedeutung hat,

gegebenenfalls in Gegenwart eines Säureakzeptors, gegebenenfalls in Gegenwart eines Verdünnungsmittels und gegebenenfalls in Gegenwart eines Katalysators umsetzt.

4. Herbizide Mittel, gekennzeichnet durch einen Gehalt an mindestens einer Verbindung der Formel (I) gemäß Anspruch 1.

5. Verwendung von Verbindungen der allgemeinen Formel (I) gemäß Anspruch 1 zur Bekämpfung von unerwünschtem Pflanzenwachstum.

6. Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

7. Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

## Claims

1. N-(4-Fluoro-phenyl)-heteroaryloxyacetamides of the general formula (I)

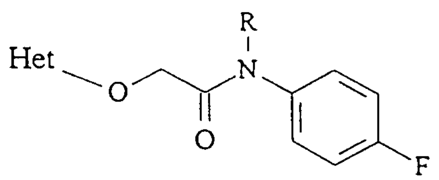

(I)

in which

R represents methyl or ethyl and

Het represents an aromatic five-membered heterocycle from the series comprising oxazolyl or thiazolyl (each of which can optionally be substituted by halogen, cyano, methyl, ethyl, n- or i-propyl, n-, i-, s- or t-butyl, difluoromethyl, dichloromethyl, trifluoromethyl or trichloromethyl), 1,2,4-oxadiazolyl, 1,3,4-oxadiazolyl, 1,2,4-thiadiazolyl or 1,3,4-thiadiazolyl (each of which can optionally be substituted by fluorine, chlorine, bromine, methyl, ethyl, n- or i-propyl, n-, i-, s- or t-butyl, trifluoromethyl, dichloromethyl, trichloromethyl, chlorodifluoromethyl, fluorodichloromethyl, methylthio, ethylthio, n- or i-propylthio, methylsulphinyl, ethylsulphinyl, n-or i-propylsulphinyl, methylsulphonyl, ethylsulphonyl, n- or i-propylsulphonyl, phenyl or fluorophenyl), benzoxazolyl or benzothiazolyl (each of which is substituted by chlorine, bromine, methyl, ethyl, n- or i-propyl, difluoromethyl, dichloromethyl, trifluoromethyl or trichloromethyl) and - in the event that R represents ethyl - also represents unsubstituted benzoxazolyl and benzothiazolyl.

2. N-(4-Fluoro-phenyl)-heteroaryloxyacetamides of the formula (I) according to Claim 1, characterized in that, in the formula,

R represents methyl or ethyl and

Het represents 1,2,4-thiadiazolyl (which is optionally substituted by chlorine, trifluoromethyl, chlorodifluoromethyl or methylsulphonyl), 1,3,4-thiadiazolyl (which is optionally substituted by chlorine, trifluoromethyl, chlorodifluoromethyl or methylsulphonyl), or benzoxazolyl or benzothiazolyl, each of which is monosubstituted by chlorine.

3. Process for the preparation of N-(4-fluoro-phenyl)heteroaryloxyacetamides of the formula (I), according to Claim 1, characterized in that heterocycles of the general formula (II)

$$\text{Het - X} \qquad \text{(II)}$$

in which

Het has the meaning given in Claim 1 and

X represents halogen or alkylsulphonyl,

are reacted with N-(4-fluoro-phenyl)-hydroxyacetamides of the general formula (III)

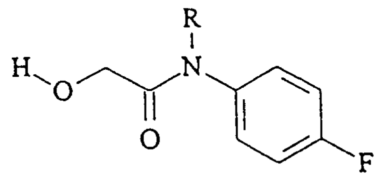

(III)

in which

R has the meaning given in Claim 1,

if appropriate in the presence of an acid acceptor, if appropriate in the presence of a diluent and if appropriate in the presence of a catalyst.

4. Herbicidal agents, characterized in that they contain at least one compound of the formula (I) according to Claim 1.

5. Use of compounds of the general formula (I) according to Claim 1 for combating undesired plant growth.

6. Method of combating weeds, characterized in that compounds of the general formula (I) according to Claim 1 are allowed to act on the weeds or their environment.

7. Process for the preparation of herbicidal agents, characterized in that compounds of the general formula (I) according to Claim 1 are mixed with extenders and/or surface-active agents.

**Revendications**

1. N-(4-fluorophényl)-hétéroaryloxyacétamides de formule générale (I)

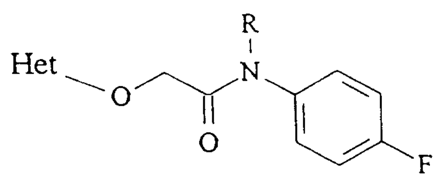

(I)

dans laquelle

R est un groupe méthyle ou éthyle et

Het désigne un hétérocycle aromatique pentagonal de la série oxazolyle ou thiazolyle (dont chacun peut être substitué le cas échéant par un halogène, un groupe cyano, méthyle, éthyle, n-propyle, isopropyle, n-butyle, isobutyle, sec.-butyle, tertio-butyle, difluorométhyle, dichlorométhyle, tifluorométhyle ou trichlorométhyle), 1,2,4-oxadiazolyle, 1,3,4-oxadiazolyle, 1,2,4-thiadiazolyle ou 1,3,4-thiadiazolyle (dont chacun peut être substitué le cas échéant par du fluor, du chlore, du brome, un groupe méthyle, éthyle, n-propyle, isopropyle, n-butyle, isobutyle, sec.-butyle, tertio-butyle, trifluorométhyle, dichlorométhyle, trichlorométhyle, chlorodifluorométhyle, fluorodichlorométhyle, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle, phényle ou fluorophényle), benzoxazolyle ou benzothiazolyle (dont chacun peut être substitué par du chlore, du brome, un groupe méthyle, éthyle, n-propyle, isopropyle, difluorométhyle, dichlorométhyle, trifluorométhyle, ou trichlorométhyle) et - au cas où R est un groupe éthyle - également benzoxazolyle ou benzothiazolyle non substitué.

2. N-(4-fluorophényl)-hétéroaryloxyacétamides de formule générale (I) suivant la revendication 1, caractérisés en ce que dans cette formule

R est un groupe méthyle ou éthyle et
Het est un groupe 1,2,4-thiadiazolyle (qui est substitué le cas échéant par du chlore, un groupe trifluorométhyle, chlorodifluorométhyle ou méthylsulfonyle), 1,3,4-thiadiazolyle (qui est substitué le cas échéant par du chlore, un groupe trifluorométhyle, chlorodifluorométhyle ou méthylsulfonyle) ou un groupe benzoxazolyle ou benzothiazolyle dont chacun est monosubstitué par du chlore.

3. Procédé de production de N-(4-fluorophényl)hétéroaryloxyacétamides de formule générale (I) suivant la revendication 1, caractérisé en ce qu'on fait réagir des hétérocycles de formule générale (II)

Het - X (II)

dans laquelle

Het a la définition indiquée dans la revendication 1 et
X est un halogène ou un groupe alkylsulfonyle,

avec des N-(4-fluorophényl)-hydroxyacétamides de formule générale (III)

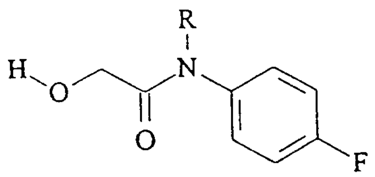

(III)

dans laquelle

R a la définition indiquée dans la revendication 1,

le cas échéant en présence d'un accepteur d'acide, éventuellement en présence d'un diluant et le cas échéant en présence d'un catalyseur.

4. Compositions herbicides, caractérisées par une teneur en au moins un composé de formule (I) suivant la revendication 1.

5. Utilisation de composés de formule générale (I) suivant la revendication 1 pour combattre une croissance non désirée de plantes.

6. Procédé pour combattre des mauvaises herbes, caractérisé en ce qu'on fait agir des composés de formule générale (I) suivant la revendication 1 sur les mauvaises herbes ou sur leur milieu.

7. Procédé de préparation de compositions herbicides, caractérisé en ce qu'on mélange des composés de formule générale (I) suivant la revendication 1 avec des diluants et/ou des agents tensio-actifs.